# FASCICULE DE BREVET EUROPEEN

(11) **EP 2 717 794 B1**
(45) Date de publication et mention de la délivrance du brevet: **15.07.2015**
(21) Numéro de dépôt: 12731030.8
(22) Date de dépôt: 21.05.2012
(51) Int. Cl.: A61B 17/80

(54) **DISPOSITIF D'OSTEOSYNTHESE A PLAQUE ET BROCHES**
OSTEOSYNTHESEVORRICHTUNG MIT PLATTE UND STIFTEN
OSTEOSYNTHESIS DEVICE WITH PLATE AND PINS

(30) Priorité: 06.06.2011 FR 1154878
(43) Date de publication de la demande: 16.04.2014
(73) Titulaire: Ateliers Laumonier, SAS, 95690 Nesles La Vallée (FR); Worcel, Julia, 95680 Montlignon (FR); Worcel, Manuel, 75012 Paris (FR); Paoli, Jean, 95300 Pontoise (FR); Paoli, Myriam, 95300 Pontoise (FR); Lombardo-Fiault, Bernard, 75011 Paris (FR); Worcel, Marie, 95680 Montlignon (FR); Paoli, Edouard, 95300 Pontoise (FR); Paoli, Albert, 95300 Pontoise (FR); Laumonier, Alain, 95690 Nesles La Vallée (FR); Laumonier, Rémi, 95690 Nesles La Vallée (FR); Laumonier, Nicolas, 95300 Pontoise (FR); Laumonier, Yves, 95690 Nesles La Vallée (FR); Laumonier, Bruno, 95690 Nesles La Vallée (FR); Worcel, Alexandre, Parc de Mongarny 95680 Montlignon (FR)
(72) Inventeur: WORCEL, Alexandre, 95680 Montlignon (FR)
(74) Mandataire: Gendron, Vincent Christian
(86) Numéro de dépôt international: PCT/FR2012/051125
(87) Numéro de publication internationale: WO 2012/168613

(56) Documents cités:
- FR-A1- 2 905 589
- US-A1- 2006 015 104
- US-A1- 2006 195 104

## Description

La présente invention se rapporte à un dispositif d'ostéosynthèse permettant de maintenir au moins deux éléments osseux, l'un par rapport à l'autre.

Des dispositifs connus comprennent des plaques, et des broches destinées à être maintenues à travers lesdites plaques au moyen d'une douille de blocage. Les plaques s'étendent longitudinalement de manière à pouvoir être ajustées le long des éléments osseux situés dans le prolongement l'un de l'autre et elles présentent au moins deux orifices taraudés venant respectivement en regard des deux éléments osseux. Les orifices taraudés traversent bien évidemment les plaques de part en part.

Quant aux douilles de blocage, elles comprennent une partie vissable et une partie d'entraînement surmontant la partie vissable. Au surplus, les deux parties sont séparées l'une de l'autre par une gorge ménagée sur la douille de blocage. La partie vissable présente un bord libre et elle est de forme générale tronconique, son diamètre décroissant à partir de la gorge précitée vers le bord libre. En outre, elle présente des fentes axiales définissant ainsi des créneaux. Lors du montage, la plaque est ajustée le long des éléments osseux, tandis que les broches sont respectivement ancrées dans ces éléments osseux à travers les orifices taraudés. Après l'ancrage, les broches présentent une partie libre résiduelle qui s'étend en saillie de la plaque et sur lesquelles on vient engager les douilles de blocage. Le diamètre interne de ces douilles correspond, au jeu fonctionnel près, au diamètre externe des broches, qui permettent de les guider en translation. La partie vissable est alors portée au niveau de l'orifice taraudé et la douille est engagée en rotation au moyen de la partie d'entraînement qui la surmonte. Lorsque la partie vissable vient en prise avec les filets de l'orifice taraudé, compte tenu de sa forme générale tronconique, les créneaux définis par les fentes tendent à se déformer concentriquement en formant des mors qui viennent progressivement enserrer la broche. À un stade ultime de l'entraînement en rotation de la partie vissable, la broche demeure en position fixe par rapport à la plaque, la partie vissable de la douille étant engagée à force entre les parois de l'orifice taraudé de la plaque et la broche. Bien évidemment, une telle opération est conduite pour les deux broches, de manière à maintenir les deux éléments osseux en position fixe l'un par rapport à l'autre.

Ensuite, au moyen d'une pince coupante engagée au droit de la gorge précitée, on cisaille à la fois la douille et la partie de broche saillante. De la sorte, la face de la plaque opposée aux éléments osseux est débarrassée des éléments saillants, c'est-à-dire la partie d'entraînement de la douille et la partie libre de la broche, tandis que la partie de broche ancrée dans les éléments osseux est solidaire de la plaque grâce à la douille de blocage qui l'enserre.

On pourra se référer au document FR 2 905 589, lequel décrit un tel dispositif d'ostéosynthèse.

Ces dispositifs sont couramment utilisés et bien qu'ils permettent une intervention rapide et un bon maintien des éléments osseux pendant la durée suffisante à leur consolidation, et ce par rapport aux générations précédentes de dispositifs d'ostéosynthèse, le besoin de dispositifs plus performants et moins coûteux à mettre en oeuvre se fait toujours sentir.

Aussi, un problème qui se pose et que vise à résoudre la présente invention est de fournir un dispositif plus économique, permettant une mise en oeuvre plus aisée et qui présente une grande fiabilité après installation.

Dans ce but, la présente invention propose un dispositif d'ostéosynthèse comprenant, d'une part une plaque apte à être ajustée le long d'un élément osseux et au moins une broche destinée à être ancrée dans ledit élément osseux à travers ladite plaque, et d'autre part une douille de blocage apte à recevoir ladite au moins une broche et permettant de solidariser ladite plaque et ladite au moins une broche, ladite douille de blocage présentant une partie vissable et une partie d'entraînement pour visser ladite partie vissable à travers ladite plaque, ladite partie vissable et ladite partie d'entraînement étant séparée l'une de l'autre axialement par une zone frangible, ladite partie vissable présentant des fentes axiales formant des créneaux entre lesdites fentes axiales, ladite partie vissable étant apte à coopérer avec ladite plaque lorsque ladite partie vissable est vissée à travers ladite plaque pour pouvoir déformer concentriquement lesdits créneaux et à enserrer ladite broche, ladite zone frangible étant destinée à être rompue pour libérer ladite partie vissable de ladite partie d'entraînement. Selon l'invention, lesdites fentes axiales s'étendent dans ladite zone frangible de manière à pouvoir fragmenter ladite partie vissable en créneaux indépendants lorsque ladite zone frangible est rompue de façon à pouvoir ajuster la position relative desdits créneaux autour de ladite au moins une broche. Une zone frangible est en effet une zone moins résistante mécaniquement, qui s'étend ici entre ladite partie vissable et ladite partie d'entraînement et dans laquelle s'opère la rupture.

Ainsi, une caractéristique d'invention réside dans la douille de blocage et plus précisément dans le ménagement de fentes axiales qui s'étendent jusque dans la zone frangible. De la sorte, lorsque la zone frangible est rompue, les créneaux de la partie vissable formant des mors, deviennent indépendants les uns des autres et les tensions radiales qui s'exercent entre la broche et la plaque, tendent à s'équilibrer dans toutes les directions sensiblement parallèles au plan moyens de la plaque. Bien qu'indépendants, les mors qui par nature de la partie vissable, présentent des portions de filet en prise dans la plaque, sont maintenus axialement en position fixe par rapport à la broche et la plaque.

En outre, les mors devenant indépendants, et non plus solidaires les uns des autres, le serrage de la broche est moins sensible à la dureté des métaux et au couple de serrage ainsi qu'à la précision d'usinage des pièces.

De plus, et ainsi qu'on l'expliquera plus en détail dans la suite de la description, si la fragmentation de la partie vissables de la douille intermédiaire permet un meilleur serrage initial, et par conséquent d'obtenir un ensemble plus rigide au moment de l'installation, cet ensemble évolue dans le temps et sa rigidité diminue. Une telle caractéristique permet de solliciter progressivement et mécaniquement les éléments osseux et par conséquent de favoriser la consolidation osseuse.

Selon une caractéristique de mise en oeuvre de l'invention particulièrement avantageuse, ladite zone frangible est auto-frangible de manière à pouvoir rompre ladite zone frangible lorsque lesdits créneaux enserrent ladite au moins une broche, tandis que ladite partie d'entraînement est entraînée à force. Ainsi, grâce à cette caractéristique il n'est nul besoin de venir cisailler la douille mais simplement la broche et au surplus, au ras de la partie vissable comme on l'expliquera par la suite dans la description. Au surplus, lorsque la partie d'entraînement est entraînée à force et que les mors enserrent la broche, la résistance de la partie vissable en rotation devient alors supérieure à la résistance mécanique de la zone frangible, laquelle tend à se déformer en cisaillement selon des directions sensiblement parallèles aux tangentes à la douille. Aussi, la résistance en rotation de chacun des créneaux de la partie vissable est différente en fonction de la position relative de la broche et de la plaque, et notamment de leur inclinaison, de sorte que la déformation en cisaillement de la zone frangible s'effectue différemment aux droits des créneaux. De la sorte, et grâce aux fentes axiales, les créneaux vont alors se rapprocher ou s'écarter circonférentiellement les uns des autres au fur et à mesure de la rupture, selon la résistance de chacun durant leur rotation. Grâce à cet ajustement relatif des créneaux les uns par rapport aux autres, les efforts radiaux entre la plaque et la broche s'équilibrent parfaitement. Les créneaux forment alors des coins indépendants entre la broche et la plaque et ils sont prisonniers de l'ensemble. Car, d'un côté les filets du créneau sont en prise dans la plaque, et de l'autre la broche est en appui à force. Aussi, la broche est parfaitement maintenue en position fixe par rapport à la plaque.

Préférentiellement, ladite douille de blocage comporte une gorge entre ladite partie vissable et ladite partie d'entraînement pour former ladite zone frangible. Cette gorge est aisément ménagée sur l'extérieur de la douille, et sa profondeur est précisément déterminée afin de calibrer l'épaisseur de douille en fond de gorge. Car en effet, cette épaisseur doit être prédéterminée en fonction du matériau, puisqu'elle doit résister suffisamment pour entraîner à force la partie vissable à travers la plaque mais aussi, se rompre lorsque le serrage des créneaux, ou mors, est suffisant pour bloquer la broche. La douille de blocage est par exemple réalisée en acier inoxydable de qualité chirurgicale.

En outre, ladite partie vissable présente un bord circulaire libre situé à l'opposé de ladite zone frangible, et le diamètre de ladite partie vissable décroit avantageusement de ladite zone frangible vers ledit bord libre circulaire. De la sorte, ladite partie vissable coopère avec ladite plaque, et plus précisément avec l'orifice taraudé dont l'enveloppe est par exemple cylindrique, lorsque ladite partie vissable est vissée à travers ladite plaque pour pouvoir déformer concentriquement lesdits créneaux. Pour ce faire, le diamètre maximal de la partie vissable doit être supérieur au diamètre de l'orifice taraudé comme on l'expliquera plus en détail dans la suite de la description. Aussi, au fur et à mesure de la rotation de la partie vissable à travers la plaque, les créneaux formant des coins s'enfoncent progressivement entre la plaque et la broche et l'enserrent.

Selon un mode de réalisation particulièrement avantageux, ladite partie vissable présente une enveloppe tronconique. Une telle forme a le mérite de pouvoir être réalisée aisément. Au surplus, le serrage de la broche est progressif à mesure que la partie vissable est entraînée en rotation. On prévoit également selon un autre mode de réalisation, un orifice taraudé de la plaque de forme tronconique.

Par ailleurs, et selon un autre mode de réalisation particulièrement avantageux, le dispositif comprend en outre une douille intermédiaire destinée à être vissée dans l'épaisseur de ladite plaque, ladite douille de blocage étant destinée à être engagée à l'intérieur de ladite douille intermédiaire. Une telle douille intermédiaire, également dénommée réducteur, permet de réaliser un meilleur ajustage et un meilleur centrage de la broche. Aussi, la plaque présente des évidements circulaires taraudés à travers lesquels viennent en prise les douilles intermédiaires. Ces dernières présentent également un taraudage intérieur formant l'orifice taraudé précité. Ensuite la douille intermédiaire peut être montée. Par ailleurs, la douille intermédiaire permet le démontage du dispositif d'ostéosynthèse après qu'il a été installé. En effet, la broche est totalement solidaire de la douille intermédiaire grâce à la mise en oeuvre de la douille de blocage dès l'installation sur l'élément osseux. Aussi, après quelques semaines, pour retirer le dispositif, la douille intermédiaire est dévissée avec la broche qui en est solidaire.

Selon une variante de réalisation préférée, ladite partie vissable présente quatre fentes axiales, diamétralement opposées deux à deux. Ainsi, lors du montage on obtient quatre créneaux, ou segments tronconiques, enserrant la broche, ce qui permet de la bloquer radialement dans toutes les directions. Elle est bien évidemment bloquée également axialement compte tenue des efforts radiaux et concentriques qui s'exercent sur elle.

En outre, ladite partie d'entraînement de ladite douille de blocage présente deux méplats diamétralement opposés permettant, au moyen d'une clé simple, de venir entraîner en rotation la partie d'entraînement. Bien évidemment, d'autres formes de la partie d'entraînement sont envisagées, et notamment des formes hexagonales.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la Figure 1 est une vue schématique en coupe axiale d'un dispositif d'ostéosynthèse conforme à l'invention en cours de montage ;
- la Figure 2 est une vue schématique de face d'un élément du dispositif d'ostéosynthèse représenté sur la Figure 1;
- la Figure 3 est une vue schématique de dessous de l'élément représenté sur la Figure 2 ; et,
- la Figure 4 est une vue schématique de dessus d'éléments du dispositif d'ostéosynthèse représenté sur la Figure 1.

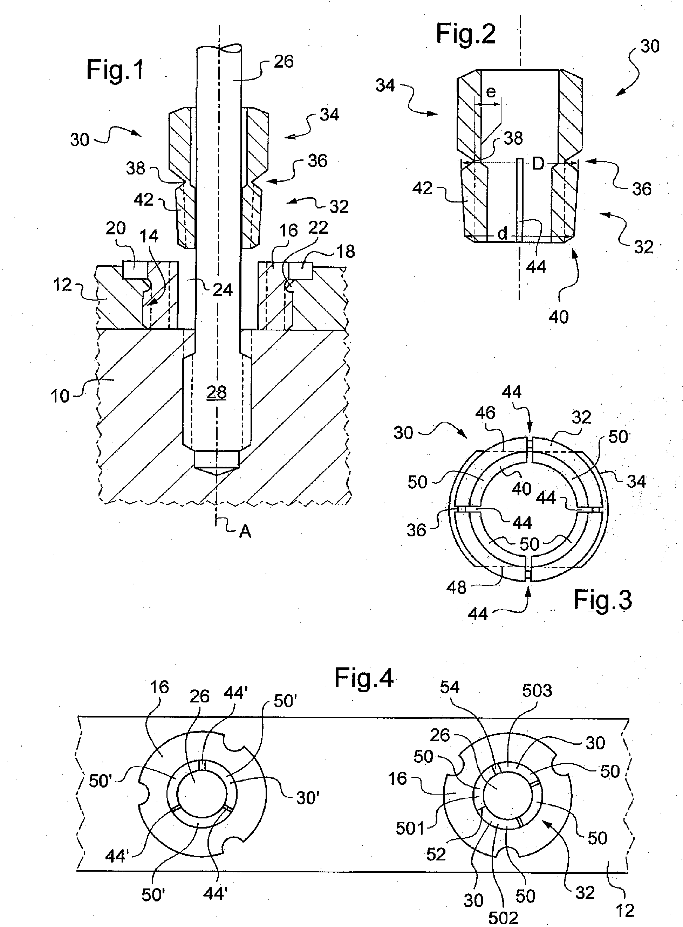

La Figure 1 illustre partiellement un élément osseux 10 sur lequel est appliquée une plaque 12. Cette dernière présente un évidement circulaire 14 taraudé et la traversant de part en part selon un axe de symétrie A. Une douille intermédiaire 16 est alors montée vissée à l'intérieur de l'évidement circulaire 14. La douille intermédiaire 16 présente une collerette 18 dans laquelle sont ménagées des encoches radiales 20 d'entraînement en rotation. De plus, la plaque 12 présente un embrèvement 22 permettant de loger à tout le moins partiellement la collerette 18. La douille intermédiaire 16 présente un orifice taraudé 24. On observera que l'orifice taraudé 24 représenté sur la Figure 1 est de symétrie cylindrique. Ainsi, la douille intermédiaire 16 est apte à être solidarisée à la plaque 12 dans son épaisseur. Selon un mode de mise en oeuvre de l'invention particulièrement avantageux, l'orifice taraudé 24 est de symétrie conique de révolution. La génératrice de l'orifice taraudé 24 et son axe de symétrie forment un angle α compris par exemple entre 3° et 7°.

Est également illustrée sur cette Figure, une broche 26 laquelle traverse la plaque 12 et l'orifice taraudé 24 et est vissée à l'intérieur de l'élément osseux 10. Pour ce faire, la broche 26 présente une extrémité auto-foreuse 28 qui a été vissée dans l'élément osseux à travers la plaque 12 et à travers une épaisseur donnée d'élément osseux en fonction des possibilités et des nécessités d'ancrage.

Aussi, le corps de la broche 26 est engagé à l'intérieur d'une douille de blocage 30, laquelle présente une partie vissable 32 orientée vers la douille intermédiaire 16 et une partie d'entraînement 34 opposée. On observera sur cette Figure 1 que tous les éléments sont agencés coaxialement vis-à-vis de l'évidement circulaire 14.

On détaillera à présent la douille de blocage 30 représentée de côté sur la Figure 2. Elle est réalisée en acier chirurgical, par exemple en acier 316 L écroui ou non. On retrouve sa partie d'entraînement 34 surmontant sa partie vissable 32. Ces deux parties sont séparées l'une de l'autre selon une direction axiale, par une gorge en V 36, formant une zone frangible. Cette gorge 36 présente un fond de gorge 38 au droit de laquelle, l'épaisseur e de la douille est comprise, en moyenne entre 0,5 et 3 mm, par exemple 1,5 mm. On peut également prévoir une épaisseur e du fond de gorge variable sur la circonférence. Par exemple des épaississements de renfort, judicieusement repartis, comme on l'expliquera ci-après.

La partie vissable 32 présente un bord inférieur libre 40 et sont diamètre maximal D, près de la gorge 36 est supérieur à son diamètre minimal d, près du bord inférieur 40. Aussi, la partie vissable 32 est de forme générale tronconique de révolution, son diamètre décroissant progressivement de la gorge 36 vers le bord inférieur libre 40. La génératrice de la partie vissable 32 et son axe de symétrie, forment ici un angle β, avantageusement supérieur à l'angle α de l'orifice taraudé 24 et compris par exemple, entre 3° et 8°. La partie vissables 32 présente également un filetage extérieur 42. On expliquera plus en détail par la suite de la description les avantages de la différence d'angle de conicité entre la partie vissable 32 et l'orifice taraudé 24.

En outre, et selon une caractéristique particulièrement avantageuse de l'invention, la partie vissable 32 de la douille de blocage 30 présente des fentes axiales 44 qui s'étendent axialement du bord inférieur 40 jusqu'à la gorge 36 dans laquelle elle débouche. Une seule de ces fentes axiales 44 apparaît sur la Figure 2. Elles sont espacées les unes des autres, par exemple de 90°.

On se reportera à présent sur la Figure 3 pour décrire plus en détail, en vue de dessous, la douille de blocage 30. On y retrouve la partie vissable 32 et son bord inférieur 40 ainsi que les fentes axiales 44 débouchant dans la gorge 36. Ces fentes axiales 44 s'étendent bien évidemment selon l'axe de la douille 30 mais aussi radialement dans l'épaisseur de la partie vissable 32. La largeur de ces fentes axiales 44 est voisine de l'épaisseur radiale de la partie vissable 32 de la douille, par exemple 0,7 fois cette épaisseur. Les fentes axiales 44 définissent par la même des créneaux 50 déformables, en forme de segments tronconiques et aptes à former des mors, lorsqu'ils sont déformés de manière concentrique. On observera ici, que les épaississements de renfort en fond de gorge peuvent être ménagés au droit des créneaux déformables, de manière à renforcer localement leur liaison avec la partie d'entraînement 34.

En arrière de la partie vissable 32 s'étend la partie d'entraînement 34 laquelle présente deux méplats diamétralement opposés 46, 48, représentés en traits interrompus. Ces méplats 46, 48 sont destinés à recevoir une clé simple, par exemple une clé plate, ou une clé à oeil adaptée, pour pouvoir entraîner en rotation la partie vissable 32 comme on l'expliquera ci-après.

On se reportera tout d'abord, une nouvelle fois à la Figure 1 pour décrire le montage du dispositif d'ostéosynthèse selon l'invention et ensuite, sur la Figure 4, pour décrire le dispositif après montage et selon deux variantes de réalisation.

Tout d'abord, on retrouve sur la douille de blocage 30 représentée sur la Figure 1, la gorge 36 séparant la partie vissable 32 de la partie d'entraînement 34 et le filetage extérieur 42. À partir de la position de la douille de blocage 30 telle que représentée sur cette Figure 1, la douille est entraînée à coulissement, guidée par la broche 26 jusqu'à la douille intermédiaire 16, et elle va être entraînée en rotation par l'intermédiaire de la partie d'entraînement 34. La partie vissable 32 vient alors en prise dans le filetage de l'orifice taraudé 24. Dans une première phase de vissage, le filetage extérieur 42 de la partie vissable 32 et le filetage de l'orifice taraudé 24 s'interpénètrent faiblement l'un dans l'autre grâce à la différence d'angle de la partie vissable 32 et de l'orifice taraudé 24. En effet, lorsque l'on vient ajuster la partie vissable 32 à l'intérieur de l'orifice taraudé 24, leurs filets viennent respectivement en prise sur une plus faible longueur que s'ils avaient la même conicité. En conséquence, dans cette première phase de vissage, la partie vissable 32 ne se déforme pas et la broche 26 demeure libre par rapport à la douille de blocage 30. De la sorte, elle peut encore être réglée si nécessaire. Au surplus, la douille de blocage 30 peut encore être dévissée pour réajuster la broche 26.

Progressivement, à mesure que la partie vissable 32 s'engage à l'intérieur de la douille intermédiaire 16, le filetage extérieur 42 et le filetage de l'orifice taraudé 24 s'interpénètrent progressivement et des forces de frottement apparaissent. Cela est dû à la forme tronconique de la partie vissable 32 et de l'orifice taraudé 24. Aussi, dans une deuxième phase de vissage, la rotation de la douille de blocage 30 provoque les mouvements concentriques des créneaux 50 qui viennent s'appuyer radialement contre le corps de la broche 26. Les créneaux 50, en segments tronconiques, forment des mors ou des coins venant coincer le corps de la broche 26 et la douille intermédiaire 16, à mesure que la douille de blocage 30 va être entraînée en rotation. Les mouvements radiaux et concentriques des créneaux 50, et en battement par rapport à la partie d'entraînement 34, sont rendus possibles grâce aux fentes axiales 44 et aussi grâce à leur flexibilité.

La forme tronconique de la partie vissable 32 avec ses diamètres maximal D et minimal d est prédéfinie avec les paramètres géométriques de l'orifice taraudé 24 tronconique et le diamètre de la broche 26, de manière à ce que la partie vissable 32 se trouve dans une position de blocage relatif lorsqu'elle se trouve logée à l'intérieur de la douille intermédiaire 16.

Dans cette situation alors, on entre dans une troisième phase de vissage, dans laquelle la douille de blocage 30 va se déformer en cisaillement au niveau du fond de gorge 38.

On comprend aisément que la broche 26 n'est pas nécessairement exactement perpendiculaire à la plaque 12 ni parfaitement cylindrique tout comme les douilles 16, 30. Aussi, dans la troisième phase de vissage, les créneaux 50, encore solidaires les uns des autres, sont entraînés en frottement à la fois contre l'intérieur de l'orifice taraudé 24 et contre l'extérieur du corps de la broche 26. Ils subissent ainsi des efforts de frottement différents pour chacun. Partant, grâce aux fentes axiales 44 qui se prolongent dans la gorge 36, la douille de blocage 30, au droit de son fond de gorge 38 et des créneaux 50, va se déformer différentiellement en cisaillement, en fonction des forces de frottement subies précisément par les créneaux 50. Aussi, parmi les créneaux 50, celui qui subira le plus de forces de frottement, provoquera une déformation plus importante de la partie de fond de gorge 38 en regard et partant, un début de cisaillement, tandis que les autres créneaux 50 demeurent entraînés en rotation jusqu'à ce qu'ils subissent à leur tour des efforts de frottement importants. De la sorte, le cisaillement de la douille de blocage 30 au niveau du fond de gorge 38 se produit au niveau des créneaux 50, successivement, à mesure qu'ils offrent de plus en plus de résistance aux mouvements de rotation. Cela permet d'entraîner successivement les créneaux 50 dans des positions où les contraintes radiales sont sensiblement équivalentes, avant que la partie d'entraînement 34 ne soit totalement désolidarisée de la partie vissable 32. De la sorte, les efforts radiaux qui s'appliquent sur le corps de la broche 26 et à l'opposé, à l'intérieur de l'orifice taraudé 24, sont uniformément répartis autour de la broche 26. Et partant, elle est parfaitement solidaire de la plaque 12.

On observera que, l'épaisseur e de la douille en fond de gorge 38 doit être, d'une part suffisante pour que la douille de blocage 30 puisse être vissée jusqu'au bout de sa course dans la douille intermédiaire 16 sans se rompre, et d'autre part, suffisamment faible pour que la douille de blocage 30 puisse se rompre précisément en bout de course. Cette position est atteinte lorsque la partie vissable 32 est entièrement logée dans l'épaisseur de la douille intermédiaire 16 et plus précisément lorsque la gorge 36 se situe au niveau de la partie supérieure de la douille intermédiaire 16.

Dans un système où la partie vissable est monobloc, seules les forces de frottement dues à la pression axiale entre les filets de vis, s'opposent au dévissage. Par contre, ici, chaque créneau 50 s'oppose individuellement au dévissage car d'une part ils présentent des bords francs aptes à venir s'ancrer dans les parois de la broche et à l'opposé dans les parois de la douille intermédiaire 16, et d'autre part ils forment coin entre la broche 26 et la douille intermédiaire 16. Leur ancrage est d'autant plus important que le couple de serrage a été grand. En outre, les créneaux 50 en formant coin agissent sur toute la longueur du filetage de la douille intermédiaire 16 alors que selon l'art antérieur, le serrage était localisé axialement et s'opérait uniquement autour de la partie déformable de la douille de blocage. Ainsi, on obtient un ensemble douille intermédiaire 16, broche 26, parfaitement rigide qui va néanmoins évoluer et se déformer au fil du temps pour le bienfait de la consolidation osseuse comme on va l'expliquer ci-après.

En effet, les créneaux 50, coincés entre la broche 26 et la douille intermédiaire 16 vont être sollicités et soumis à des contraintes qui s'exercent sur l'élément osseux 10 et partant sur la plaque 12 et la broche 26. Aussi, les créneaux 50 vont, au fil du temps, c'est-à-dire au bout de quelques semaines, se déformer par écrouissage par exemple et également s'user par frottement. En conséquence, la liaison entre la broche 26 et la douille intermédiaire 16, et donc la plaque 12, va devenir de moins en moins rigide tout en restant solidaire l'un de l'autre. Ainsi donc, à l'origine le montage de la broche 26 et de la douille intermédiaire 16 et très rigide, et il permet ainsi une reprise rapide des fonctions des membres qu'il consolide avec une diminution des douleurs postopératoires. Ensuite, au cours du temps, il est apte à se déformer plus aisément ce qui permet un transfert progressif des contraintes mécaniques sur les éléments osseux au bénéfice de leur consolidation. De la sorte, le dispositif d'ostéosynthèse selon l'invention est mécaniquement évolutif vers une moindre rigidification et permet donc de dynamiser progressivement l'ossification.

Après que la partie d'entraînement 34 soit rendue libre de la partie vissable 32, on vient, au moyen d'une pince coupante cisailler la broche 26 précisément au ras de la partie vissable 32 et de la douille intermédiaire 16.

Ainsi, on obtient une vis dont la tige formée par une portion de broche et la tête, formée de la douille intermédiaire 16 et de la partie vissable 32 de la douille de blocage 30, sont solidarisés in situ. De la sorte, la longueur de tige utile est adaptée en fonction de la profondeur de vissage de la broche à l'intérieur de l'élément osseux, et la position relative de la plaque 12 et de la douille intermédiaire 16.

On observera que le centre de pivotement de la broche 26 par rapport à la douille intermédiaire 16 se situe au niveau de l'extrémité cisaillée de la broche 26, les créneaux 50 demeurant intacts, tandis que la broche 26 tend à être entraînée en battements du côté de l'élément osseux 10. C'est alors de ce côté de l'élément osseux 10, que les créneaux 50 tendent à s'écrouir.

En outre, la plaque 12 et la broche 26 sont totalement isolées l'une de l'autre et sont maintenues l'une à l'autre par l'intermédiaire des créneaux 50, lesquels encaissent l'échappement des contraintes. Aussi, si par exemple une broche a un appui plus important dans une partie corticale au regard des autres broches vissées dans des parties spongieuses, les créneaux 50 encaisseront en premier les différences de contraintes. Cela permettra d'équilibrer les contraintes dans le dispositif.

On se reportera à présent sur la Figure 4 montrant en vue de dessus, la plaque 12 avec deux broches 26 espacées l'une de l'autre et maintenues à l'intérieur d'une douille intermédiaire 16 au moyen d'une douille de blocage 30 telle qu'illustrée sur les Figures 2 et 3, pour l'une des broches, et une autre douille de blocage 30' selon une variante de réalisation pour l'autre des broches. Cette autre douille de blocage 30' présente non plus quatre fentes axiales, mais simplement trois 44'.

On observera tout d'abord la douille de blocage 30 telle que décrite ci-dessus, et plus précisément les quatre créneaux 50 formés par sa partie vissable 32. En effet, durant la troisième phase de vissage, la rotation des créneaux 50 a eu lieu selon des amplitudes différentes en fonction des frottements. Aussi, parmi les créneaux 50, certains 501, 502 sont venus en contact l'un avec l'autre au niveau d'un joint de contact 52 en faisant disparaître l'espace de la fente 44 qui les séparait, tandis que certains autres, 501, 503 se sont écartés l'un de l'autre en agrandissant l'espace 54 de la fente 44 qui les séparait.

S'agissant de l'autre douille de blocage 30' pour laquelle seules trois fentes 44' ont été ménagées, on observera, de la même manière que les espaces définis par les fentes se sont réduits, ou bien se sont agrandis durant la troisième phase de vissage.

Par ailleurs, selon un mode de mise en oeuvre de l'invention non représenté, quatre fentes axiales sont ménagées dans la partie vissable de la douille de blocage, mais seulement deux d'entre elles, diamétralement opposées, débouchent dans la zone frangible. Aussi, les créneaux sont divisés en deux paires de créneaux accolés. De la sorte, les deux créneaux accolés sont déformables l'un par rapport à l'autre tandis que les deux paires de créneaux sont indépendantes l'une de l'autre autour de la broche. Une telle configuration, offre d'autres possibilités de pose.

## Revendications

1. Dispositif d'ostéosynthèse comprenant, d'une part une plaque (12) apte à être ajustée le long d'un élément osseux (10) et au moins une broche (26) destinée à être ancrée dans ledit élément osseux (10) à travers ladite plaque (12), et d'autre part une douille de blocage (30) apte à recevoir ladite au moins une broche (26) et permettant de solidariser ladite plaque (12) et ladite au moins une broche (26), ladite douille de blocage (30) présentant une partie vissable (32) et une partie d'entraînement (34) pour visser ladite partie vissable à travers ladite plaque (12), ladite partie vissable (32) et ladite partie d'entraînement (34) étant séparées l'une de l'autre axialement par une zone frangible (38), ladite partie vissable présentant des fentes axiales (44) formant des créneaux (50) entre lesdites fentes axiales, ladite partie vissable (32) étant apte à coopérer avec ladite plaque (12) lorsque ladite partie vissable est vissée à travers ladite plaque pour pouvoir déformer concentriquement lesdits créneaux (50) et à enserrer ladite broche (26), ladite zone frangible (38) étant destinée à être rompue pour libérer ladite partie vissable (32) de ladite partie d'entraînement (34) ;
**caractérisé en ce que** lesdites fentes axiales (44) s'étendent dans ladite zone frangible (38) de manière à pouvoir fragmenter ladite partie vissable (32) en créneaux indépendants (50) lorsque ladite zone frangible (38) est rompue de façon à pouvoir ajuster la position relative desdits créneaux (50) autour de ladite au moins une broche (26).

2. Dispositif d'ostéosynthèse selon la revendication 1, **caractérisé en ce que** ladite zone frangible (38) est auto-frangible de manière à pouvoir rompre ladite zone frangible lorsque lesdits créneaux (50) enserrent ladite au moins une broche (26), tandis que ladite partie d'entraînement (34) est entraînée à force.

3. Dispositif d'ostéosynthèse selon la revendication 1 ou 2, **caractérisé en ce que** ladite douille de blocage (30) comporte une gorge (36) entre ladite partie vissable (32) et ladite partie d'entraînement (34) pour former ladite zone frangible (38).

4. Dispositif d'ostéosynthèse selon l'une quelconque des revendications 1 à 3, **caractérisé en ce que** ladite partie vissable (32) présente un bord circulaire libre (40) situé à l'opposé de ladite zone frangible (38), et **en ce que** le diamètre de ladite partie vissable (32) décroit de ladite zone frangible (38) vers ledit bord libre circulaire (40).

5. Dispositif d'ostéosynthèse selon la revendication 4, **caractérisé en ce que** ladite partie vissable (32) présente une enveloppe tronconique de révolution, ladite partie vissable (32) présentant une génératrice formant un angle β avec son axe de symétrie.

6. Dispositif d'ostéosynthèse selon l'une quelconque des revendications 1 à 5, **caractérisé en ce qu'**il comprend en outre une douille intermédiaire (16) destinée à être vissée dans l'épaisseur de ladite plaque (12), ladite douille de blocage (30) étant destinée à être engagée à l'intérieur de ladite douille intermédiaire (16).

7. Dispositif d'ostéosynthèse selon l'une quelconque des revendications 1 à 6, **caractérisé en ce que** ladite douille intermédiaire (16) présente un orifice taraudé (24) de symétrie tronconique de révolution, ledit orifice taraudé (24) présentant une génératrice formant un angle α avec son axe de symétrie.

8. Dispositif d'ostéosynthèse selon les revendications 5 et 7, **caractérisé en ce que** l'angle β de ladite partie vissable (32) est supérieur à l'angle α dudit orifice taraudé (24).

9. Dispositif d'ostéosynthèse selon l'une quelconque des revendications 1 à 8, **caractérisé en ce que** ladite partie vissable (32) présente quatre fentes axiales (44), diamétralement opposées deux à deux.

10. Dispositif d'ostéosynthèse selon l'une quelconque des revendications 1 à 9, **caractérisé en ce que** ladite partie d'entraînement (34) de ladite douille de blocage (30) présente deux méplats diamétralement opposés (46, 48).

## Patentansprüche

1. Osteosynthesevorrichtung umfassend einerseits eine Platte (12), die entlang eines Knochenteils (10) ausgerichtet werden kann, und mindestens einen Stift (26), der bestimmt ist, in dem Knochenelement (10) durch die Platte (12) hindurch verankert zu werden, und andererseits eine Verriegelungshülse (30),die ausgebildet ist, den mindestens einen Stift (26) aufzunehmen und die Platte (12) und den zumindest einen Stift (26) zu verbinden, wobei die Verriegelungshülse (30) einen Gewindeabschnitt (32) und einen Antriebsabschnitt (34) zum Einschrauben des Gewindeabschnitts in die Platte (12) aufweist, wobei der Gewindeabschnitt (32) und der Antriebsabschnitt (34) axial voneinander durch einen fragilen Bereich (38) getrennt sind, wobei der Gewindeabschnitt axiale Schlitze (44) aufweist, welche Vorsprünge (50) zwischen den axialen Schlitzen bilden, wobei der Gewindeabschnitt (32) angepasst ist zum Zusammenwirken mit der Platte (12), wenn der Gewindeabschnitt in die Platte eingeschraubt ist, um die Vorsprünge (50) konzentrisch verformen zu können und den Stift (26) zu umschließen, wobei der fragile Bereich (38) dazu bestimmt ist, gebrochen zu werden, um den Gewindeabschnitt (32) von dem Antriebsabschnitt (34) freizustellen;
**dadurch gekennzeichnet, dass** sich die axialen Schlitze (44) in den fragilen Bereich (38) erstrecken, um den Gewindeabschnitt (32) in unabhängige Vorsprünge (50) aufteilen zu können, wenn der fragile Bereich (38) gebrochen ist, um die relative Position der Vorsprünge (50) um den zumindest einen Stift einstellen zu können.

2. Osteosynthesevorrichtung nach Anspruch 1, **dadurch gekennzeichnet, dass** der fragile Bereich (38) selbstzerbrechend ist, um den fragilen Bereich brechen zu können, wenn die Vorsprünge (50) den zumindest einen Stift (26) umschließen, während der Antriebsabschnitt (34) kraftbetätigt wird.

3. Osteosynthesevorrichtung nach Anspruch 1 oder 2, **dadurch gekennzeichnet, dass** die Verriegelungshülse (30) eine Nut (36) zwischen dem Gewindeabschnitt (32) und dem Antriebsabschnitt (34) aufweist, um den fragilen Bereich (38) zu bilden.

4. Osteosynthesevorrichtung nach einem der Ansprüche 1 bis 3, **dadurch gekennzeichnet, dass** der Gewindeabschnitt (32) einen kreisförmigen freien Rand (40) gegenüberliegend dem fragilen Bereich (38) aufweist und der Durchmesser des Gewindeabschnitts (32) sich von dem fragilen Bereich (38) in Richtung des kreisförmigen freien Rands (40) verringert.

5. Osteosynthesevorrichtung nach Anspruch 4, **dadurch gekennzeichnet, dass** der Gewindeabschnitt (32) eine rotationskegelstumpfförmige Hülle aufweist, wobei der Gewindeabschnitt (32) eine Mantellinie aufweist, die einen Winkel β mit seiner Symmetrieachse bildet.

6. Osteosynthesevorrichtung nach einem der Ansprüche 1 bis 5, **dadurch gekennzeichnet, dass** sie ferner eine Zwischenhülse (16) umfasst, die bestimmt ist, in der Dicke der Platte (12) eingeschraubt zu werden, wobei die Verriegelungshülse (30) bestimmt ist, mit dem Innenraum der Zwischenhülse (16) in Eingriff gebracht zu werden.

7. Osteosynthesevorrichtung nach einem der Ansprüche 1 bis 6, **dadurch gekennzeichnet, dass** die Zwischenhülse (16) eine rotationskegelstumpfförmigsymmetrische Gewindebohrung (24) aufweist, wobei die Gewindebohrung (24) eine Mantelllinie aufweist, die einen Winkel α mit ihrer Symmetrieachse bildet.

8. Osteosynthesevorrichtung nach einem der Ansprüche 5 und 7, **dadurch gekennzeichnet, dass** der Winkel β des Gewindeabschnitts (32) größer als der Winkel α der Gewindebohrung (24) ist.

9. Osteosynthesevorrichtung nach einem der Ansprüche 1 bis 8, **dadurch gekennzeichnet, dass** der Gewindeabschnitt (32) vier jeweils paarweise diametral einander gegenüberliegende axiale Schlitze (44) aufweist.

10. Osteosynthesevorrichtung nach einem der Ansprüche 1 bis 9, **dadurch gekennzeichnet, dass** der Antriebsabschnitt (34) der Verriegelungshülse (30) zwei diametral gegenüberliegende Abflachungen (46, 48) aufweist.

## Claims

1. Osteosynthesis device comprising, firstly, a plate (12) which can be adjusted along a bone element (10), and at least one pin (26) which is intended to be anchored in said bone element (10) through said plate (12), and, secondly, a locking bushing (30), capable of receiving said at least one pin (26) and making it possible to rigidly connect said plate (12) and said at least one pin (26), said locking bushing (30) having a screwable part (32) and a drive part (34) for screwing said screwable part through said plate (12), a breakable zone (38) axially separating said screwable part (32) and said drive part (34), said screwable part having axial slots (44) that form rings (50) between said axial slots, said screwable part (32) being capable of cooperating with said plate (12) when said screwable part is screwed through said plate so as to be able to concentrically deform said rings (50) and to clamp said pin (26), said breakable zone (38) being intended to be broken in order to free said screwable part (32) from said drive part (34); **characterised in that** said axial slots (44) extend into said breakable zone (38) so as to be able to fragment said screwable part (32) into independent rings (50) when said breakable zone (38) is broken, so as to be able to adjust the relative position of said rings (50) around said at least one pin (26).

2. Osteosynthesis device according to claim 1, **characterised in that** said breakable zone (38) is self-breakable so as to be able to break said breakable zone when said rings (50) clamp said at least one pin (26), whereas said drive part (34) is driven by force.

3. Osteosynthesis device according to either claim 1 or claim 2, **characterised in that** said locking bushing (30) comprises a groove (36) between said screwable part (32) and said drive part (34) in order to form said breakable zone (38).

4. Osteosynthesis device according to any of claims 1 to 3, **characterised in that** said screwable part (32) has a free circular edge (40) situated opposite said breakable zone (38), and **in that** the diameter of said screwable part (32) decreases from said breakable zone (38) towards said free circular edge (40).

5. Osteosynthesis device according to claim 4, **characterised in that** said screwable part (32) has a frustoconical envelope of revolution, said screwable part (32) having a generatrix that forms an angle β with the axis of symmetry thereof.

6. Osteosynthesis device according to any of claims 1 to 5, **characterised in that** it further comprises an intermediate bushing (16) which is intended to be screwed into the thickness of said plate (12), said locking bushing (30) being intended to be engaged inside said intermediate bushing (16).

7. Osteosynthesis device according to any of claims 1 to 6, **characterised in that** said intermediate bushing (16) has a tapped orifice (24) of frustoconical symmetry of revolution, said tapped orifice (24) having a generatrix that forms an angle α with the axis of symmetry thereof.

8. Osteosynthesis device according to claims 5 and 7, **characterised in that** the angle β of said screwable part (32) is greater than the angle α of said tapped orifice (24).

9. Osteosynthesis device according to any of claims 1 to 8, **characterised in that** said screwable part (32) has four axial slots (44) which are diametrically opposed to one another in pairs.

10. Osteosynthesis device according to any of claims 1 to 9, **characterised in that** said drive part (34) of said locking bushing (30) has two diametrically opposed flat portions (46, 48).
